# EUROPEAN PATENT SPECIFICATION

(11) **EP 4 494 577 B1**
(45) Date of publication and mention of the grant of the patent: **23.09.2026**
(21) Application number: 23829902.8
(22) Date of filing: 09.06.2023
(51) Int. Cl.: A61B 17/22, A61B 17/12, A61L 31/06, A61L 31/14

(54) **CLOSURE IMPLANT AND METHOD FOR PREPARING SAME**
VERSCHLUSSIMPLANTAT UND VERFAHREN ZUR HERSTELLUNG DAVON
IMPLANT DE FERMETURE ET SON PROCÉDÉ DE PRÉPARATION

(30) Priority: 30.06.2022 CN 202210763980
(43) Date of publication of application: 22.01.2025
(73) Proprietor: Shanghai MicroPort Medical (Group) Co., Ltd., Shanghai 201203 (CN)
(72) Inventor: GU, Jialei, Shanghai 201203 (CN); LU, Jun, Shanghai 201203 (CN); LIU, Wei, Shanghai 201203 (CN); WANG, Xueqin, Shanghai 201203 (CN); YUE, Bin, Shanghai 201203 (CN)
(74) Representative: Marks & Clerk LLP
(86) International application number: PCT/CN2023/099317
(87) International publication number: WO 2024/001718

(56) References cited:
- CN-A- 103 006 286
- CN-A- 103 110 444
- CN-A- 104 739 478
- CN-A- 107 205 736
- CN-A- 107 773 283
- CN-A- 110 464 403
- CN-A- 112 641 484
- CN-A- 115 054 307
- JP-A- 2008 539 807

## Description

### TECHNICAL FIELD

The present disclosure relates to the technical field of medical devices, and in particular to an occlusion implant and a method for preparing the same.

### BACKGROUND

A spring coil is an occlusion implant for arterial fistulas and aneurysms, and is also commonly used for emergency closing of blood vessels when the blood vessels are accidentally injured and bleeding during surgery. A mechanism of action of the spring coil is to reduce a local blood flow velocity and promote thrombus formation, thereby achieving an effect of occlusion.

Common spring coils are mainly divided into bare metal spring coils, hydrogel metal spring coils, and metal spring coils with polymer fluff. Although the common spring coils have a mature and effective occlusion effect, because metal cannot be degraded, the metal may still remain in a body to form a mass effect after the arterial fistulas or aneurysms heal, and compression on other blood vessels or nerves cannot be relieved. In this regard, although degradable spring coils have been developed in the field, current degradable spring coil technologies are not mature enough, and there is still a risk of blood vessel or aneurysm recanalization.

Related technologies are known from CN112641484A, CN107205736A, and CN110464403A.

### SUMMARY

According to various embodiments of the present disclosure, the present disclosure provides an occlusion implant and a method for preparing the same. The present invention is set out in the appended claims.

An occlusion implant, including:
a first coil, the first coil being formed by weaving a woven material, the woven material including at least a first wire, and the first wire being an electrospun yarn prepared by electrospinning;
a second coil, the second coil being arranged inside the first coil, and the second coil including a developing material and a polymer material; and
an anti-unwinding component, the anti-unwinding component being connected to at least one of the first coil and the second coil, wherein the anti-unwinding component (200) is formed by an anti-unwinding filament, the anti-unwinding filament is folded in half, so that two ends of the anti-unwinding filament are merged together, the merged end being referred to as a distal end of the anti-unwinding filament; and the anti-unwinding filament forms a ring-shaped structure, which is referred to as a proximal end of the anti-unwinding filament; the distal end of the anti-unwinding filament is threaded along an interior of the first coil (100) from the proximal end to the distal end, the distal end of the anti-unwinding filament is fixed to a distal end (130) of the first coil (100), and the ring-shaped structure is formed outside a proximal end (120) of the first coil (100).

In an embodiment, at least one of a proximal end and a distal end of the first coil is closed.

In an embodiment, the woven material includes at least a first wire and a second wire, and the first wire and the second wire have different degradation rates.

In an embodiment, the first wire is made of a material selected from at least one of poly-L-lactic acid and polycaprolactone, and the second wire is made of a material selected from at least one of polydioxanone, poly-DL-lactic acid, and polyglycolic acid.

In an embodiment, at least part of the anti-unwinding component is arranged inside the second coil.

In an embodiment, the anti-unwinding component includes an anti-unwinding section and a connecting section, the anti-unwinding section is located inside the second coil, and the connecting section is located outside a proximal end of the second coil.

In an embodiment, the anti-unwinding component is made of a degradable material.

In an embodiment, the anti-unwinding component is made of a material selected from at least one of polydioxanone, poly-DL-lactic acid, polyglycolic acid, poly-L-lactic acid, poly lactic-co-glycolic acid, polycaprolactone, and poly-p-dioxanone.

In an embodiment, a mass ratio of the developing material to the polymer material in the second coil is about 1:2 to 4: 1.

In an embodiment, the polymer material in the second coil includes a degradable filament, and the degradable filament is wound into a spiral coil. A ratio of a coil pitch of the degradable filament to a diameter of the degradable filament ranges from 1: 1 to 4: 1.

In an embodiment, the polymer material in the second coil includes a non-degradable filament, and the non-degradable filament is wound into a spiral coil. A ratio of a coil pitch of the non-degradable filament to a diameter of the non-degradable filament ranges from 2:1 to 8:1.

A method for preparing an occlusion implant, including the following steps:
dissolving a degradable polymer material in a solvent to obtain a homogeneous solution, preparing a spun film by electrospinning, stretching the spun film to form an electrospun yarn, and weaving the electrospun yarn to form a first coil;
preparing a second coil with a developing material and a polymer material, and arranging the second coil inside the first coil; and
shaping the first coil and the second coil, and connecting an anti-unwinding component to at least one of the first coil and the second coil.

In an embodiment, the method includes the following step:
closing at least one of a proximal end and a distal end of the first coil by means of hot-melt closing.

In an embodiment, the method includes the following steps:
applying a hot-melted degradable material to at least one of the proximal end and the distal end of the first coil, and then cooling the degradable material to close at least one of the proximal end and the distal end of the first coil.

In an embodiment, the method includes the following steps:
placing at least one of the proximal end and the distal end of the first coil in a mold, hot-melting at least one of poly-p-dioxanone, poly-DL-lactic acid, polyglycolic acid, poly-L-lactic acid, poly lactic-co-glycolic acid, polycaprolactone, or polydioxanone in the mold, and then cooling to close at least one of the proximal end and the distal end of the first coil.

Details of one or more embodiments of the present disclosure are set forth in the following accompanying drawings and descriptions. Other features, objectives, and advantages of the present disclosure will become obvious with reference to the specification, the accompanying drawings, and the claims.

### BRIEF DESCRIPTION OF THE DRAWINGS

In order to more clearly illustrate the technical solutions in the embodiments of the present disclosure or the conventional art, the accompanying drawings used in the description of the embodiments or the conventional art will be briefly introduced below. It is apparent that, the accompanying drawings in the following description are only some embodiments of the present disclosure, and other drawings can be obtained by those of ordinary skill in the art from the provided drawings without creative efforts.
FIG. 1 is a schematic cross-sectional view of a first part of an occlusion implant according to some embodiments of the present invention.
FIG. 2 is a schematic cross-sectional view of a second part of the occlusion implant according to some embodiments of the present invention.

Reference signs:
100: first coil; 200: anti-unwinding component; 300: second coil;
110: unit coil layer; 120: proximal end of first coil; 130: distal end of first coil;
210: anti-unwinding section; 220: connecting section.

### DETAILED DESCRIPTION

The technical solutions in the embodiments of the present disclosure will be described clearly and completely below with reference to the accompanying drawings in the embodiments of the present disclosure. The described embodiments are merely some of rather than all of the embodiments of the present disclosure.

In order to make the objectives, technical solutions and advantages of the embodiments of the present disclosure clearer, implementations of the present disclosure will be described below in detail with reference to the accompanying drawings. In the following description, many specific details are set forth in order to fully understand the present disclosure. However, the present disclosure can be implemented in many other ways different from those described herein.

In order to describe a structure of an occlusion implant more clearly, the term "distal end" herein is defined to represent an end away from an operator during a surgical operation, and the term "proximal end" represents an end close to the operator during the surgical operation. Unless defined otherwise, all technical and scientific terms used in the present disclosure have the same meanings as would generally understood by those skilled in the technical field of the present disclosure. The terms used in the specification of the present disclosure are for the purpose of describing specific embodiments only, and are not intended to limit the present disclosure.

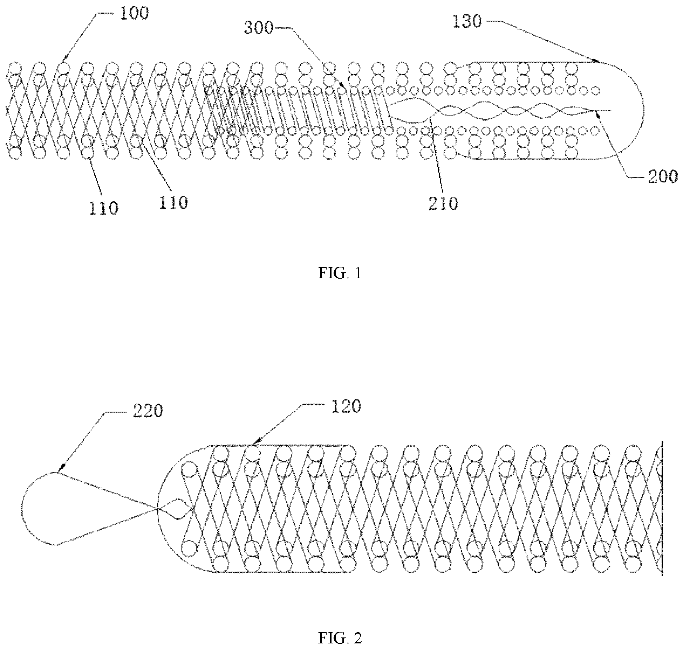

Referring to FIG. 1 and FIG. 2, an embodiment of the present disclosure provides an occlusion implant. The occlusion implant includes a first coil 100, a second coil 300, and an anti-unwinding component 200. The first coil 100 is formed by weaving a woven material. The woven material includes at least a first wire. The first wire is an electrospun yarn prepared by electrospinning. The second coil 300 is arranged inside the first coil 100. The second coil includes a developing material and a polymer material. The anti-unwinding component is connected to at least one of the first coil and the second coil. The occlusion implant includes materials used to occlude arterial fistulas or aneurysms, for example, structures such as medical spring coils, occlusive meshes, etc. When the occlusion implant is a medical spring coil, the first coil 100 and the second coil 300 of the occlusion implant are spiral coil structures formed along a spiral shape. When the occlusion implant is a medical occlusive mesh, the first coil 100 and the second coil 300 of the occlusion implant may be mesh coils formed along a mesh structure or coil structures formed along other trajectories or arc surfaces. Those skilled in the art may select the structure of the occlusion implant according to an actual requirement, which is not limited herein.

The first coil 100 may include at least one unit coil layer 110, and the unit coil layer 110 is formed by weaving at least one woven material. For example, the first coil 100 may be formed by stacking a plurality of unit coil layers 110. Since each unit coil layer 110 is made of the woven material, the first coil 100 is also made of the woven material. Interiors of the first coil 100 and the second coil 300 may be understood as structures such as inner rings of the first coil 100 and the second coil 300 or gaps between the coils, which are not limited herein.

The first coil 100 of the occlusion implant is a main part of the occlusion implant that performs an occluding function. The first coil 100 is formed by weaving through a weaving process, and the woven material includes a first wire. A surface of the first coil 100 formed by weaving the first wire through the weaving process has a rough texture and structure. The rough texture and structure can speed up formation of thrombus, contribute to a good thrombogenic effect, promote the rapid formation of thrombus in a short term, effectively reduce packing density at positions such as arterial fistulas and aneurysms, improve occlusion efficiency, reduce an amount of the occlusion implant, and reduce high pressure of blood vessel wall or tumor wall during surgery. Moreover, the rough texture and structure may produce a porous structure. The porous structure can support growth and attachment of cells, which may be used as a cell growth scaffold in a medium and long term so as to speed up endothelialization and promote lesion healing. This can effectively reduce the risk of blood vessel or aneurysm recanalization.

It is to be noted that the weaving process of forming the first coil 100 by weaving may be any weaving manner. For example, the weaving process may be double-strand weaving with one strand pressed against one strand, or three-strand weaving with one strand pressed against two strands, or four-strand weaving with two strands pressed against two strands, or four-strand weaving with one strand pressed against three strands, or other common weaving manners. In the first coil 100 formed by weaving, a spacing between repeating woven units may be ranged from 0.5 to 10 times a diameter of the wire. Those skilled in the art may select an appropriate weaving manner according to a requirement, so as to form a rough texture and structure, which is not limited herein.

A proximal end 120 of the first coil or a distal end 130 of the first coil may be closed. After at least one of the proximal end 120 of the first coil and the distal end 130 of the first coil is closed, an occlusion effect on blood vessels can be achieved. The closing may be carried out in a variety of manners. For example, the proximal end 120 of the first coil and the distal end 130 of the first coil are closed by means of hot-melt closing. In the case of hot-melt closing, a biodegradable material such as poly-p-dioxanone (PPDO), poly-DL-lactic acid (PDLLA), polyglycolic acid (PGA), poly-L-lactic acid (PLLA), poly lactic-co-glycolic acid (PLGA), polycaprolactone (PCL), or polydioxanone (PDO) is hot melted in a mold, and then cooled to close the proximal end 120 of the first coil and the distal end 130 of the first coil. Such a closing manner can further improve softness of the proximal end 120 of the first coil or the distal end 130 of the first coil, forming a relatively soft end structure, so that the proximal end 120 of the first coil or the distal end 130 of the first coil may not damage or puncture the blood vessels when in contact with the blood vessel wall.

In addition to including the first wire, the woven material for forming the first coil 100 by weaving can weave by combining the first wire with another second wire. For example, in an embodiment, the woven material includes at least a first wire and a second wire. The first wire and the second wire may be selected as degradable materials with different degradation rates. Therefore, the first coil 100 has a degradable effect and can reduce a long-term mass effect. Moreover, the first wire and the second wire in the woven material have different degradation rates, enabling the first coil 100 to form a relay degradation effect in the body. That is, the first wire and the second wire in the woven material degrade successively at different degradation rates, which can shorten a recovery period of a patient and reduce risks of occlusion failure and blood flow recanalization.

The first wire and the second wire have different degradation rates. For example, in an embodiment, a material of the first wire may include a degradable material with a degradation rate of 2 to 4 years, and a material of the second wire may include a degradable material with a degradation rate of 1.5 years. Therefore, when the occlusion implant remains in the body within 1.5 years, the woven wire may be degraded first, while the first wire has not been completely degraded. The degradation of the second wire can reduce the mass effect, and retention of the first wire can continue to perform the occluding function and reduce the risks of occlusion failure and blood flow recanalization. Therefore, through two different degradation rates, the risk of recanalization due to the mass effect can be balanced and a good therapeutic effect can be achieved.

In an embodiment, the material of the first wire includes any one or any combination of poly-L-lactic acid and polycaprolactone, and the material of the second wire includes any one or any combination of polydioxanone, poly-DL-lactic acid, and polyglycolic acid. In addition, those skilled in the art may also select another material according to a requirement, which is not limited herein.

The anti-unwinding component 200 can prevent straightening of the first coil 100 under an external force, thereby achieving an anti-unwinding effect. The anti-unwinding component 200 may adopt any structural form. For example, as shown in FIG. 1 and FIG. 2, in an embodiment, the anti-unwinding component 200 includes an anti-unwinding section 210 and a connecting section 220. The anti-unwinding section 210 is located inside the first coil 100, and the connecting section 220 is located outside the proximal end 120 of the first coil. The anti-unwinding component 200 may be formed by one or more anti-unwinding filaments. For example, one anti-unwinding filament may be folded in half. Two ends of the anti-unwinding filament are merged together, which may be referred to as a distal end of the anti-unwinding filament. The anti-unwinding filament may be seen on the right in FIG. 1. In this case, the anti-unwinding filament may form a ring-shaped structure on the left in FIG. 2, which may be referred to as a proximal end of the anti-unwinding filament. The distal end of the anti-unwinding filament may be threaded along the interior of the first coil 100 from the proximal end to the distal end, the distal end of the anti-unwinding filament may be fixed to the distal end 130 of the first coil, and a ring-shaped structure is formed outside the proximal end 120 of the first coil by using the proximal end of the anti-unwinding filament. A length of the ring-shaped structure may range from 0.5 mm to 3 mm. A part of the anti-unwinding component 200 located inside the first coil 100 may constitute the anti-unwinding section 210, and an exposed part of the ring-shaped structure of the anti-unwinding component 200 may constitute the connecting section 220.

The anti-unwinding component 200 may be made of a degradable material or a non-degradable material. For example, in an embodiment, the material of the anti-unwinding component 200 may include any one or any combination of degradable materials such as polydioxanone, poly-DL-lactic acid, polyglycolic acid, poly-L-lactic acid, poly lactic-co-glycolic acid, polycaprolactone, and poly-p-dioxanone.

In an embodiment, the anti-unwinding component may be connected to the first coil or to the second coil. The anti-unwinding component may be detachably connected or non-detachably connected, for example, fixedly connected, buckled, or bonded, which is not limited therein. Moreover, by means of an assembly relationship between the first coil 100 and the second coil 300, at least part of the anti-unwinding component 200 may alternatively be arranged inside the first coil 100.

The material of the second coil 300 may be selected according to a functional requirement. For example, in an embodiment, the material of the second coil 300 is a degradable material, or the material of the second coil 300 is a non-degradable material, or the material of the second coil 300 includes a developing material and a polymer material. The polymer material may be a degradable material or a non-degradable material.

It is to be noted that "the material of the second coil 300 includes a developing material and a polymer material", it may be understood as that the material of the second coil 300 includes a layer structure formed by the developing material and a layer structure formed by the polymer material. For example, the layer structure formed by the developing material may be arranged on an inner layer or an outer layer of the layer structure formed by the polymer material, and the layer structure formed by the developing material and the layer structure formed by the polymer material may be one or more layers, so that the layer structure formed by the developing material and the layer structure formed by the polymer material are spaced apart with each other in various arrangements.

In addition, "the material of the second coil 300 includes a developing material and a polymer material", it may also be understood as that the material of the second coil 300 is formed by blending and extrusion molding the developing material and the polymer material. The developing material and the polymer material may be mixed according to a certain proportion. The developing material may be an iodine-based contrast agent, barium sulfate, tantalum powder, or the like.

For example, in an embodiment, the second coil 300 is formed by weaving a degradable filament into a spiral coil structure, and a ratio of a coil pitch of the second coil 300 to a diameter of the degradable filament ranges from 1:1 to 4:1. For example, the ratio of the coil pitch of the second coil 300 to the diameter of the degradable filament is 1:1, 2:1, 3:1, 4:1, or the like. Alternatively, in an embodiment, the second coil 300 is formed by weaving a non-degradable filament into a spiral coil structure, and a ratio of a coil pitch of the second coil 300 to a diameter of the non-degradable filament ranges from 2:1 to 8:1. For example, the ratio of the coil pitch of the second coil 300 to the diameter of the non-degradable filament is 2:1, 3:1, 4:1, 5:1, 6:1, 7:1, 8:1, or the like. Alternatively, the second coil 300 is formed by the developing material and the polymer material, and a mass ratio of the developing material to the polymer material in the second coil 300 ranges from 1:2 to 4:1. For example, the ratio of the developing material to the polymer material in the second coil 300 is 1:2, 1:1, 2:1, 3:1, 4:1, or the like, which may be set by those skilled in the art according to a requirement, and is not limited herein.

The mass ratio of the developing material to the polymer material is suitable for the second coil 300 whose material includes a layer structure formed by the developing material and a layer structure formed by the polymer material. Alternatively, the mass ratio of the developing material to the polymer material is also suitable for the second coil 300 whose material is formed by blending and extrusion molding the developing material and the polymer material.

The present disclosure further provides a method for preparing an occlusion implant, including the following steps: dissolving a degradable polymer material in a solvent to obtain a homogeneous solution, preparing a spun film by electrospinning, stretching the spun film to form an electrospun yarn, and weaving the electrospun yarn to form a first coil 100;
preparing a second coil 300 with a developing material and a polymer material, and arranging the second coil 300 inside the first coil 100; and
shaping the first coil 100 and the second coil 300, and connecting an anti-unwinding component 200 to at least one of the first coil 100 and/or the second coil 300.

In an embodiment, the first coil 100 is formed by weaving through a weaving process, and the woven material includes a first wire. A surface of the first coil 100 formed by the first wire through a weaving process has a rough texture and structure. The rough texture and structure can speed up formation of thrombus, contribute to a good thrombogenic effect, promote the rapid formation of thrombus in a short term, effectively reduce packing density at positions such as arterial fistulas and aneurysms, improve occlusion efficiency, reduce an amount of the occlusion implant, and reduce high pressure of blood vessel wall or tumor wall during surgery. Moreover, the rough texture and structure may produce a porous structure. The porous structure can support growth and attachment of cells, which may be used as a cell growth scaffold in a medium and long term so as to speed up endothelialization and promote lesion healing. This can effectively reduce the risk of blood vessel or aneurysm recanalization.

In an embodiment, the first wire is prepared through the following steps: dissolving a degradable polymer material in a solvent to obtain a homogeneous solution, performing electrospinning with a collection apparatus to obtain a first wire film, and utilizing the electrospun film to form the first wire. When the first wire is prepared through the above steps, various process parameters during the preparation may be defined to obtain the expected first wire.

For example, a weight average molecular weight of the degradable polymer may be defined to a range from 5 W to 20 W. For example, the weight average molecular weight of the degradable polymer may be defined to 5 W, 6 W, 7 W, 8 W, 9 W, 10 W, 11 W, 12 W, 13 W, 14 W, 15 W, 16 W, 17 W, 18 W, 19 W, 20 W, or the like.

The solvent may be defined to include any one or any combination of dichloromethane, chloroform, acetone, ethyl acetate, dimethylacetamide, dimethyl sulfoxide, or hexafluoroisopropanol.

A concentration of the homogeneous solution may be defined to a range from 2% to 15%. For example, the concentration of the homogeneous solution may be defined to 2%, 3%, 4%, 5%, 6%, 7%, 8%, 9%, 10%, 11%, 12%, 13%, 14%, 15%, or the like. The spinning may be operated at a defined spinning voltage ranged from 5 kV to 30 kV. For example, the spinning voltage in the spinning operation may be defined to 5 kV, 6 kV, 7 kV, 8 kV, 9 kV, 10 kV, 11 kV, 12 kV, 13 kV, 14 kV, 15 kV, 16 kV, 17 kV, 18 kV, 19 kV, 20 kV, 21 kV, 22 kV, 23 kV, 24 kV, 25 kV, 26 kV, 27 kV, 28 kV, 29 kV, or 30 kV.

The spinning may be operated at a defined injection rate ranged from 0.005 ml/min to 0.05 ml/min. For example, the injection rate in the spinning operation may be defined to 0.005 ml/min, 0.01 ml/min, 0.015 ml/min, 0.02 ml/min, 0.025 ml/min, 0.03 ml/min, 0.035 ml/min, 0.04 ml/min, 0.045 ml/min, 0.05 ml/min, or the like.

The spinning may be operated at a defined injection translation speed ranged from 50 mm/min to 5000 mm/min. For example, the injection translation speed in the spinning operation may be defined to 50 mm/min, 300 mm/min, 500 mm/min, 800 mm/min, 1000 mm/min, 2000 mm/min, 3000 mm/min, 4000 mm/min, 5000 mm/min, or the like.

The spinning may be operated at a defined temperature ranged from 15°C to 35°C. For example, the temperature in the spinning operation may be defined to 15°C, 20°C, 25°C, 30°C, or 35°C.

The spinning may be operated at a defined humidity ranged from 25% to 45%. For example, the humidity in the spinning operation may be defined to 25%, 30%, 35%, 40%, 45%, or the like.

A thickness of the electrospun film may be defined to a range from 1 µm to 10 µm. For example, the thickness of the electrospun film may be defined to 1 µm, 2 µm, 3 µm, 4 µm, 5 µm, 6 µm, 7 µm, 8 µm, 9 µm, or 10 µm.

A fiber diameter of the electrospun film may be defined to a range from 300 nm and 2000 nm. For example, the fiber diameter of the electrospun film may be defined to 300 nm, 500 nm, 800 nm, 1000 nm, 1300 nm, 1500 nm, 1800 nm, or 2000 nm. At the same time, the electrospun film may have a one-layer structure or a multi-layer structure. When the electrospun film has a multi-layer structure, different layers of the electrospun film may have different fiber diameters.

A diameter of the first wire may be defined to a range from 0.02 mm to 0.1 mm. For example, the diameter of the first wire may be defined to 0.02 mm, 0.03 mm, 0.04 mm, 0.05 mm, 0.06 mm, 0.07 mm, 0.08 mm, 0.09 mm, or 0.1 mm.

The collection apparatus may be selected as a collection cylinder with a certain speed and diameter and with a release capability. For example, the speed of the collection cylinder may range from 10 rpm to 2000 rpm, and the diameter may range from 2 cm to 30 cm. According to a requirement, the collection cylinder may obtain the release capability by disposing a release coating or laying a release film or release paper. After the electrospun film is formed through the collection cylinder, the electrospun film may be removed, cut into suitable sizes and shapes, stretched and twisted with a stretching and twisting device at a certain temperature, finally forming the first wire with a diameter of 0.02 mm to 0.1 mm. In the stretching and twisting process, the temperature may be kept at a range from 50°C to 100°C, a stretching degree may be defined to 200% to 1000%, and a twisting degree may be defined to 1 turn/mm to 5 turns/mm.

In addition, the collection apparatus may also be a collection cage or a yarn collection port to replace the collection cylinder. In this case, if the collection apparatus is a yarn collection port, subsequent stretching and twisting of the electrospun film may be omitted.

After the above preparation process is completed, by using a suitable mold, the first coil 100 or the second coil 300 may be shaped at a shaping temperature of 50°C to 100°C, and then cooled down at a temperature of -20°C to 25°C for 5 min to 60 min to finally complete the shaping.

In an embodiment, the first coil 100 is formed by weaving a woven material including at least the first wire and the second wire, and the first wire and the second wire have different degradation rates. Two ends of the anti-unwinding filament are threaded along the interior of the first coil 100 from the proximal end to the distal end, the two ends of the anti-unwinding filament are fixed to the distal end 130 of the first coil, and a ring-shaped structure is formed outside the proximal end 120 of the first coil by using the anti-unwinding filament.

### Example 1

Poly-L-lactic acid with a weight average molecular weight of 12 W was dissolved in hexafluoroisopropanol to form a homogeneous solution with a mass concentration of 9%. A first wire was prepared with the homogeneous solution according to the following process parameters:
A spinning voltage was 12 kV, an injection rate was 0.015 ml/min, and an injection translation speed was 400 mm/min. A surface of the collection cylinder was coated with a silicone oil release film for release, a diameter of the collection cylinder was 12 mm, a rotate speed of the collection cylinder was 120 rpm, a collection distance was 15 cm, a temperature was 25°C, a humidity was 45%, and a spinning time was 4 min. A thickness of the electrospun film was about 2 µm, and a fiber diameter measured by scanning electron microscopy was about 1500 nm.

The silicone oil release film loaded with the electrospun film was removed from the collection cylinder and cut into strips of 2 cm. The electrospun film was peeled off and was stretched to 400% and twisted to 2.5 turns/mm with a stretching and twisting device, to obtain a first poly-L-lactic acid wire yarn with a diameter of 0.09 mm.

0.07 mm of a polylactic acid (PLA) wire was threaded through a platinum-tungsten alloy coil (i.e., the second coil 300) with a wire diameter of 0.03 mm, a pitch of 0.09 mm, and an outer diameter of 0.30 mm twice, and a connecting ring was reserved at one end. One unit coil layer 110 of the first coil 100 was formed by weaving 0.06 mm of a polyglycolic acid (PGA) wire around the platinum-tungsten alloy coil in a manner where one strand pressed one strand and a spacing between repeating units was 2 times a wire diameter. On this basis, another unit coil layer 110 of the first coil 100 was formed by weaving the above first poly-L-lactic acid wire yarn in a manner where one strand pressed one strand and the spacing between repeating units was 2 times the wire diameter. The first coil 100 having two unit coil layers 110 with an outer diameter of 0.45 mm was obtained.

Ends of the first coil 100 were closed in a mold by using molten poly-p-dioxanone (PPDO) with a molecular weight of 8 W. The first coil 100 was wound on a mold with a preset shape and shaped at 90°C for 15 min, and then cooled to room temperature, thereby completing the preparation of the first coil 100.

### Example 2

An iohexol powder and poly-L-lactic acid with a weight average molecular weight of 12 W in a mass ratio of 1.6:1 were blended, melted, extruded, and drawn into uniform fibers with a diameter of about 0.08 mm. The fibers were wound into a developable polymer coil with an external pitch of 0.10 mm and an outer diameter of 0.30 mm.

Poly-L-lactic acid with a weight average molecular weight of 12 W was dissolved in hexafluoroisopropanol to form 9% and 4% of two homogeneous solutions with different mass concentrations. A first wire was prepared with the 9% homogeneous solution according to the following process parameters:
A spinning voltage was 19 kV, an injection rate was 0.018 ml/min, and an injection translation speed was 400 mm/min. A surface of the collection cylinder was coated with polytetrafluoroethylene for release, a diameter of the collection cylinder was 12 mm, a rotate speed of the collection cylinder was 120 rpm, a collection distance was 15 cm, a temperature was 25°C, a humidity was 45%, and a spinning time was 4 min. A thickness of the electrospun film obtained was about 2 µm, and a fiber diameter measured by scanning electron microscopy was about 1200 nm. Next, the 4% homogeneous solution was spun under a collection distance adjusted to 10 cm, a temperature of 34°C, and a humidity of 40% for 8 min to obtain an electrospun film with a thickness of about 2.5 µm, and about 650 nm of a fiber diameter measured by scanning electron microscopy.

The electrospun film was removed from the collection cylinder and cut into strips of 2 cm, and was stretched to 800% and twisted to 4 turns/mm with the stretching and twisting device, to obtain a first poly-L-lactic acid wire yarn with a diameter of 0.035 mm.

0.04 mm of a polydioxanone (PDO) wire was threaded through the developable polymer coil (i.e., the second coil 300) twice, and a connecting ring was reserved at one end. One unit coil layer 110 of the first coil 100 was formed by weaving the 0.04 mm of polydioxanone (PDO) wire around the developable polymer coil in a manner where one strand pressed one strand and a spacing between repeating units was 2 times a wire diameter. On this basis, additional three unit coil layers 110 were formed by weaving the above first poly-L-lactic acid wire yarn three times in a manner where one strand pressed one strand and the spacing between repeating units was 2 times the wire diameter. The first coil 100 having four unit coil layers 110 with an outer diameter of 0.35 mm was obtained.

Ends of the first coil 100 were closed in a mold by using molten poly-p-dioxanone (PPDO) with a molecular weight of 5 W. The first coil 100 was wound on a mold with a preset shape and shaped at 70°C for 15 min, and then cooled to room temperature, thereby completing the preparation of the first coil 100.

### Example 3

Poly-L-lactic acid with a weight average molecular weight of 12 W was dissolved in hexafluoroisopropanol to form 9% and 4% of two homogeneous solutions with different mass concentrations. A first wire was prepared with the 9% homogeneous solution according to the following process parameters:
A spinning voltage was 19 kV, an injection rate was 0.018 ml/min, and an injection translation speed was 400 mm/min. A surface of the collection cylinder was coated with a silicone oil release film for release, a diameter was 12 mm, a rotate speed was 120 rpm, a collection distance was 15 cm, a temperature was 25°C, a humidity was 45%, and a spinning time was 2 min. A thickness of the electrospun film obtained was about 1.2 µm, and a fiber diameter measured by scanning electron microscopy was about 1200 nm. Next, the 4% homogeneous solution was spun under a collection distance adjusted to 10 cm, a temperature of 34°C, and a humidity of 40% for 16 min to obtain an electrospun film with a thickness of about 4 µm and about 650 nm of a fiber diameter measured by scanning electron microscopy.

The silicone oil release film loaded with the electrospun film was removed from the collection cylinder and cut into strips of 2 cm. The electrospun film was peeled off and was stretched to 800% and twisted to 3 turns/mm with the stretching and twisting device, to obtain a first poly-L-lactic acid wire yarn with a diameter of 0.035 mm.

0.04 mm of a polydioxanone (PDO) wire was threaded through a platinum-tungsten alloy coil (i.e., the second coil 300) with a wire diameter of 0.03 mm, a pitch of 0.09 mm, and an outer diameter of 0.20 mm twice, and a connecting ring was reserved at one end. One unit coil layer 110 of the first coil 100 was formed by weaving one strand of the 0.04 mm polydioxanone (PDO) wire and two strands of the above first poly-L-lactic acid wire yarns around the platinum-tungsten alloy coil in a manner where one strand pressed two strands and a spacing between repeating units was 3 to 5 times a wire diameter. On this basis, additional three unit coil layers 110 of the first coil 100 were formed by weaving one strand of the above polydioxanone (PDO) wire and one strand of the above first poly-L-lactic acid wire yarn three times in a manner where one strand pressed one strand and the spacing between repeating units was 2 times the wire diameter. The first coil 100 having four unit coil layers 110 with an outer diameter of 0.30 mm to 0.40 mm was obtained.

Ends of the first coil 100 were closed in a mold by using molten poly-p-dioxanone (PPDO) with a molecular weight of 5W. The first coil 100 was wound on a mold with a preset shape and shaped at 70°C for 15 min, and then cooled to room temperature, thereby completing the preparation of the first coil 100.

### Example 4

Polycaprolactone with a weight average molecular weight of 8 W was dissolved in hexafluoroisopropanol to form a 9% homogeneous solution. A first wire was prepared with the 9% homogeneous solution according to the following process parameters:
A spinning voltage was 13 kV, an injection rate was 0.015 ml/min, and an injection translation speed was 400 mm/min. A surface of the collection cylinder was coated with a silicone oil release film for release, a diameter was 12 mm, a rotate speed was 130 rpm, a collection distance was 15 cm, a temperature was 25°C, a humidity was 45%, and a spinning time was 4 min. A thickness of the electrospun film was about 2 µm, and a fiber diameter measured by scanning electron microscopy was about 1300 nm.

The silicone oil release film loaded with the electrospun film was removed from the collection cylinder and cut into strips of 2 cm. The electrospun film was peeled off and was stretched to 1000% and twisted to 5 turns/mm with the stretching and twisting device, to obtain a first polycaprolactone wire yarn with a diameter of 0.035 mm.

0.04 mm of a polydioxanone (PDO) wire was threaded through a platinum-tungsten alloy coil (i.e., the second coil 300) with a wire diameter of 0.03 mm, a pitch of 0.09 mm, and an outer diameter of 0.20 mm twice, and a connecting ring was reserved at one end. Two unit coil layers 110 of the first coil 100 were formed by weaving the 0.04 mm of polydioxanone (PDO) wire around the platinum-tungsten alloy coil twice in a manner where one strand pressed one strand and a spacing between repeating units was 2 times a wire diameter. On this basis, additional two unit coil layers 110 of the first coil 100 were formed by weaving the above first polycaprolactone wire yarn in a manner where one strand pressed one strand and the spacing between repeating units was 2 times the wire diameter, to obtain a first coil 100 having four unit coil layers 110 with an outer diameter of 0.35 mm.

Ends of the first coil 100 were closed in a mold by using molten poly-p-dioxanone (PPDO) with a molecular weight of 5W. The first coil 100 was wound on a mold with a preset shape and shaped at 50°C for 15 min, and then cooled to room temperature, thereby completing the preparation of the first coil 100.

## Claims

1. An occlusion implant, comprising:
a first coil (100), the first coil (100) being formed by weaving a woven material, the woven material comprising at least a first wire, and the first wire being an electrospun yarn prepared by electrospinning;
a second coil (300), the second coil (300) being arranged inside the first coil (100), and the second coil (300) comprising a developing material and a polymer material; and
an anti-unwinding component (200), the anti-unwinding component (200) being connected to at least one of the first coil (100) and the second coil (300),
**characterized in that**:
the anti-unwinding component (200) is formed by an anti-unwinding filament, the anti-unwinding filament is folded in half, so that two ends of the anti-unwinding filament are merged together, the merged end being referred to as a distal end of the anti-unwinding filament; and the anti-unwinding filament forms a ring-shaped structure, which is referred to as a proximal end of the anti-unwinding filament; the distal end of the anti-unwinding filament is threaded along an interior of the first coil (100) from the proximal end to the distal end, the distal end of the anti-unwinding filament is fixed to a distal end (130) of the first coil (100), and the ring-shaped structure is formed outside a proximal end (120) of the first coil (100).

2. The occlusion implant according to claim 1, wherein at least one of the proximal end (120) and the distal end (130) of the first coil (100) is closed.

3. The occlusion implant according to claim 1, wherein the woven material comprises at least the first wire and a second wire, and the first wire and the second wire have different degradation rates.

4. The occlusion implant according to claim 3, wherein the first wire is made of a material selected from at least one of poly-L-lactic acid and polycaprolactone, and the second wire is made of a material selected from at least one of polydioxanone, poly-DL-lactic acid and polyglycolic acid.

5. The occlusion implant according to claim 1, wherein at least part of the anti-unwinding component (200) is arranged inside the second coil (300).

6. The occlusion implant according to claim 5, wherein the anti-unwinding component (200) comprises an anti-unwinding section (210) and a connecting section (220), the anti-unwinding section (210) is located inside the second coil (300), and the connecting section (220) is located outside a proximal end (120) of the second coil (300).

7. The occlusion implant according to claim 1, wherein the anti-unwinding component (200) is made of a degradable material.

8. The occlusion implant according to claim 7, wherein the anti-unwinding component (200) is made of a material selected from at least one of polydioxanone, poly-DL-lactic acid, polyglycolic acid, poly-L-lactic acid, poly lactic-co-glycolic acid, polycaprolactone, or poly-p-dioxanone.

9. The occlusion implant according to claim 1, wherein a mass ratio of the developing material to the polymer material in the second coil (300) is about 1:2 to 4:1.

10. The occlusion implant according to claim 1, wherein the polymer material in the second coil (300) comprises a degradable filament, the degradable filament is wound into a spiral coil, and a ratio of a coil pitch of the degradable filament to a diameter of the degradable filament ranges from 1:1 to 4:1.

11. The occlusion implant according to claim 1, wherein the polymer material in the second coil (300) comprises a non-degradable filament, the non-degradable filament is wound into a spiral coil, and a ratio of a coil pitch of the non-degradable filament to a diameter of the non-degradable filament ranges from 2:1 to 8:1.

12. A method for preparing the occlusion implant according to claim 1, comprising the following steps:
dissolving a degradable polymer material in a solvent to obtain a homogeneous solution, preparing a spun film by electrospinning, stretching the spun film to form an electrospun yarn, and weaving the electrospun yarn to form a first coil (100);
preparing a second coil (300) with a developing material and a polymer material, and arranging the second coil (300) inside the first coil (100); and
shaping the first coil (100) and the second coil (300), and connecting an anti-unwinding component (200) to at least one of the first coil (100) and the second coil (300).

13. The method according to claim 12, comprising the following step:
closing at least one of the proximal end (120) and the distal end (130) of the first coil (100) by means of hot-melt closing.

14. The method according to claim 13, comprising the following steps:
applying a hot-melted degradable material to at least one of the proximal end (120) and the distal end (130) of the first coil (100), and then cooling the degradable material to close at least one of the proximal end (120) and the distal end (130) of the first coil (100).

15. The method according to claim 14, comprising the following steps:
placing at least one of the proximal end (120) and the distal end (130) of the first coil (100) in a mold, hot-melting at least one of poly-p-dioxanone, poly-DL-lactic acid, polyglycolic acid, poly-L-lactic acid, poly lactic-co-glycolic acid, polycaprolactone or polydioxanone in the mold, and then cooling to close at least one of the proximal end (120) and the distal end (130) of the first coil (100).

## Patentansprüche

1. Okklusionsimplantat, umfassend:
eine erste Spule (100), wobei die erste Spule (100) durch Weben eines Gewebematerials gebildet wird, wobei das Gewebematerial mindestens einen ersten Draht umfasst und der erste Draht ein durch Elektrospinnen hergestelltes elektrogesponnenes Garn ist;
eine zweite Spule (300), wobei die zweite Spule (300) innerhalb der ersten Spule (100) angeordnet ist und die zweite Spule (300) ein Entwicklungsmaterial und ein Polymermaterial umfasst; und
eine Anti-Abwickelkomponente (200), wobei die Anti-Abwickelkomponente (200) mit mindestens einer der ersten Spule (100) und der zweiten Spule (300) verbunden ist,
**dadurch gekennzeichnet, dass**:
die Anti-Abwickelkomponente (200) aus einem Anti-Abwickelfilament gebildet ist, wobei das Anti-Abwickelfilament in der Mitte gefaltet ist, sodass zwei Enden des Anti-Abwickelfilaments miteinander verschmolzen sind, wobei das verschmolzene Ende als distales Ende des Anti-Abwickelfilaments bezeichnet wird; und das Anti-Abwickelfilament eine ringförmige Struktur bildet, die als proximales Ende des Anti-Abwickelfilaments bezeichnet wird; das distale Ende des Anti-Abwickelfilaments entlang eines Inneren der ersten Spule (100) vom proximalen zum distalen Ende gefädelt wird, wobei das distale Ende des Anti-Abwickelfilaments an einem distalen Ende (130) der ersten Spule (100) befestigt ist und die ringförmige Struktur außerhalb eines proximalen Endes (120) der ersten Spule (100) gebildet wird.

2. Okklusionsimplantat nach Anspruch 1, wobei mindestens eines des proximalen Endes (120) und des distalen Endes (130) der ersten Spule (100) geschlossen ist.

3. Okklusionsimplantat nach Anspruch 1, wobei das Gewebematerial mindestens den ersten Draht und den zweiten Draht umfasst und der erste Draht und der zweite Draht unterschiedliche Abbaugeschwindigkeiten aufweisen.

4. Okklusionsimplantat nach Anspruch 3, wobei der erste Draht aus einem Material gefertigt ist, das aus mindestens einem von Poly-L-Milchsäure und Polycaprolacton ausgewählt ist, und der zweite Draht aus einem Material gefertigt ist, das aus mindestens einem von Polydioxanon, Poly-DL-Milchsäure und Polyglykolsäure ausgewählt ist.

5. Okklusionsimplantat nach Anspruch 1, wobei mindestens ein Teil der Anti-Abwickelkomponente (200) innerhalb der zweiten Spule (300) angeordnet ist.

6. Okklusionsimplantat nach Anspruch 5, wobei die Anti-Abwickelkomponente (200) einen Anti-Abwickelabschnitt (210) und einen Verbindungsabschnitt (220) umfasst, der Anti-Abwickelabschnitt (210) sich innerhalb der zweiten Spule (300) befindet und der Verbindungsabschnitt (220) sich außerhalb eines proximalen Endes (120) der zweiten Spule (300) befindet.

7. Okklusionsimplantat nach Anspruch 1, wobei die Anti-Abwickelkomponente (200) aus einem abbaubaren Material gefertigt ist.

8. Okklusionsimplantat nach Anspruch 7, wobei die Anti-Abwickelkomponente (200) aus einem Material gefertigt ist, das ausgewählt ist aus mindestens einem von Polydioxanon, Poly-DL-Milchsäure, Polyglykolsäure, Poly-L-Milchsäure, Polymilchsäure-co-Glykolsäure, Polycaprolacton oder Poly-p-Dioxanon.

9. Okklusionsimplantat nach Anspruch 1, wobei ein Massenverhältnis des Entwicklungsmaterials zum Polymermaterial in der zweiten Spule (300) etwa 1:2 bis 4:1 beträgt.

10. Okklusionsimplantat nach Anspruch 1, wobei das Polymermaterial in der zweiten Spule (300) ein abbaubares Filament umfasst, das abbaubare Filament zu einer spiralförmigen Spule gewickelt ist und ein Verhältnis einer Steigung der Spule des abbaubaren Filaments zu einem Durchmesser des abbaubaren Filaments im Bereich von 1:1 bis 4:1 liegt.

11. Okklusionsimplantat nach Anspruch 1, wobei das Polymermaterial in der zweiten Spule (300) ein nicht abbaubares Filament umfasst, das nicht abbaubare Filament zu einer spiralförmigen Spule gewickelt ist und ein Verhältnis einer Steigung der Spule des nicht abbaubaren Filaments zu seinem Durchmesser des nicht abbaubaren Filaments im Bereich von 2:1 bis 8:1 liegt.

12. Verfahren zur Herstellung des Okklusionsimplantats nach Anspruch 1, umfassend die folgenden Schritte:
Auflösen eines abbaubaren Polymermaterials in einem Lösungsmittel, um eine homogene Lösung zu erhalten, Herstellen eines Spinnfilms durch Elektrospinnen, Strecken des Spinnfilms zum Bilden eines Elektrospinngarns und Verweben des Elektrospinngarns zum Bilden einer ersten Spule (100);
Herstellen einer zweiten Spule (300) mit einem Entwicklungsmaterial und einem Polymermaterial und Anordnen der zweiten Spule (300) innerhalb der ersten Spule (100); und
Formen der ersten Spule (100) und der zweiten Spule (300) und Verbinden einer Anti-Abwickelkomponente (200) mit mindestens einer der ersten Spule (100) und der zweiten Spule (300).

13. Verfahren nach Anspruch 12, umfassend den folgenden Schritt:
Verschließen mindestens eines des proximalen Endes (120) und des distalen Endes (130) der ersten Spule (100) mittels Heißschmelzverschließen.

14. Verfahren nach Anspruch 13, umfassend die folgenden Schritte:
Aufbringen eines heißgeschmolzenen, abbaubaren Materials auf mindestens eines des proximalen Endes (120) und des distalen Endes (130) der ersten Spule (100) und anschließendes Abkühlen des abbaubaren Materials, um mindestens eines des proximalen Endes (120) und des distalen Endes (130) der ersten Spule (100) zu verschließen.

15. Verfahren nach Anspruch 14, umfassend die folgenden Schritte:
Einlegen mindestens eines des proximalen Endes (120) und des distalen Endes (130) der ersten Spule (100) in eine Form, Heißschmelzen mindestens eines von Poly-p-dioxanon, Poly-DL-Milchsäure, Polyglykolsäure, Poly-L-Milchsäure, Polymilchsäure-co-Glykolsäure, Polycaprolacton oder Polydioxanon in der Form und anschließendes Abkühlen zum Verschließen mindestens eines des proximalen Endes (120) und des distalen Endes (130) der ersten Spule (100).

## Revendications

1. Implant d'occlusion, comprenant :
un premier enroulement (100), le premier enroulement (100) étant formé en tissant un matériau tissé, le matériau tissé comprenant au moins un premier fil, et le premier fil étant un fil électrofilé préparé par électrofilage ;
un second enroulement (300), le second enroulement (300) étant agencé à l'intérieur du premier enroulement (100), et le second enroulement (300) comprenant un matériau de développement et un matériau polymère ; et
un composant anti-déroulement (200), le composant anti-déroulement (200) étant relié à au moins l'un du premier enroulement (100) et du second enroulement (300),
**caractérisé en ce que** :
le composant anti-déroulement (200) est formé par un filament anti-déroulement, le filament anti-déroulement est plié en deux, de sorte que deux extrémités du filament anti-déroulement soient fusionnées, l'extrémité fusionnée étant appelée extrémité distale du filament anti-déroulement ; et le filament anti-déroulement forme une structure annulaire, appelée extrémité proximale du filament anti-déroulement ; l'extrémité distale du filament anti-déroulement est enfilée à l'intérieur du premier enroulement (100) de l'extrémité proximale à l'extrémité distale, l'extrémité distale du filament anti-déroulement est fixée à une extrémité distale (130) du premier enroulement (100), et la structure annulaire est formée à l'extérieur d'une extrémité proximale (120) du premier enroulement (100).

2. Implant d'occlusion selon la revendication 1, dans lequel au moins l'une de l'extrémité proximale (120) et de l'extrémité distale (130) du premier enroulement (100) est fermée.

3. Implant d'occlusion selon la revendication 1, dans lequel le matériau tissé comprend au moins le premier fil et un second fil, et le premier fil et le second fil présentent des vitesses de dégradation différentes.

4. Implant d'occlusion selon la revendication 3, dans lequel le premier fil est composé d'au moins un matériau sélectionné parmi l'acide poly-L-lactique et la polycaprolactone, et le second fil est composé d'au moins un matériau sélectionné parmi l'acide poly-L-lactique, l'acide poly-DL-lactique et l'acide polyglycolique.

5. Implant d'occlusion selon la revendication 1, dans lequel au moins une partie du composant anti-déroulement (200) est agencée à l'intérieur du second enroulement (300).

6. Implant d'occlusion selon la revendication 5, dans lequel le composant anti-déroulement (200) comprend une section anti-déroulement (210) et une section de liaison (220), la section anti-déroulement (210) est située à l'intérieur du second enroulement (300), et la section de liaison (220) est située à l'extérieur d'une extrémité proximale (120) du second enroulement (300).

7. Implant d'occlusion selon la revendication 1, dans lequel le composant anti-déroulement (200) est composé d'un matériau dégradable.

8. Implant d'occlusion selon la revendication 7, dans lequel le composant anti-déroulement (200) est composé d'au moins un matériau sélectionné parmi la polydioxanone, l'acide poly-DL-lactique, l'acide polyglycolique, l'acide poly-L-lactique, l'acide polylactique-co-glycolique, la polycaprolactone ou la poly-p-dioxanone.

9. Implant d'occlusion selon la revendication 1, dans lequel un rapport massique du matériau de développement au matériau polymère dans le second enroulement (300) est d'environ 1:2 à 4:1.

10. Implant d'occlusion selon la revendication 1, dans lequel le matériau polymère dans le second enroulement (300) comprend un filament dégradable, le filament étant enroulé en spirale, et un rapport du pas de bobinage du filament dégradable à un diamètre du filament dégradable est compris dans la plage de 1:1 à 4:1.

11. Implant d'occlusion selon la revendication 1, dans lequel le matériau polymère du second enroulement (300) comprend un filament non dégradable, le filament étant enroulé en spirale, et un rapport du pas de bobinage du filament non dégradable à un diamètre du filament non dégradable est compris dans la plage de 2:1 à 8:1.

12. Procédé de préparation de l'implant d'occlusion selon la revendication 1, comprenant les étapes suivantes :
la dissolution d'un matériau polymère dégradable dans un solvant pour obtenir une solution homogène, la préparation d'un film filé par électrofilage, l'étirage du film filé pour former un fil électrofilé et le tissage du fil électrofilé pour former un premier enroulement (100) ;
la préparation d'un second enroulement (300) avec un matériau de développement et un matériau polymère, et l'agencement du second enroulement (300) à l'intérieur du premier enroulement (100) ; et
la mise en forme du premier enroulement (100) et du second enroulement (300), et la liaison d'un composant anti-déroulement (200) à au moins l'un du premier enroulement (100) et du second enroulement (300).

13. Procédé selon la revendication 12, comprenant l'étape suivante :
la fermeture d'au moins l'une de l'extrémité proximale (120) et de l'extrémité distale (130) du premier enroulement (100) au moyen d'une fermeture par fusion à chaud.

14. Procédé selon la revendication 13, comprenant les étapes suivantes :
l'application d'un matériau dégradable fondu à chaud sur au moins l'une de l'extrémité proximale (120) et de l'extrémité distale (130) du premier enroulement (100), puis le refroidissement du matériau dégradable pour fermer au moins l'une de l'extrémité proximale (120) et de l'extrémité distale (130) du premier enroulement (100).

15. Procédé selon la revendication 14, comprenant les étapes suivantes :
le placement d'au moins l'une de l'extrémité proximale (120) et de l'extrémité distale (130) du premier enroulement (100) dans un moule, la fusion à chaud d'au moins un élément parmi la poly-p-dioxanone, l'acide poly-DL-lactique, l'acide polyglycolique, l'acide poly-L-lactique, l'acide polylactique-co-glycolique, la polycaprolactone ou la polydioxanone dans le moule, puis le refroidissement pour fermer au moins l'une de l'extrémité proximale (120) et de l'extrémité distale (130) du premier enroulement (100).
